# EUROPÄISCHE PATENTSCHRIFT

(11) **EP 1 563 809 B1**
(45) Veröffentlichungstag und Bekanntmachung des Hinweises auf die Patenterteilung: **01.10.2014**
(21) Anmeldenummer: 05075508.1
(22) Anmeldetag: 02.08.2000
(51) Int. Cl.: A61F 2/44

(54) **Zwischenwirbel-Nucleus-Prothese und chirurgisches Verfahren zu seiner Implantation**
INTERVERTEBRAL NUCLEUS PROSTHESIS AND SURGICAL PROCEDURE FOR IMPLANTING THE SAME.
PROTHESE A NOYAU INTERVERTEBRAL ET PROCEDE CHIRURGICAL POUR L'IMPLANTER.

(30) Priorität: 03.08.1999 FR 9910167
(43) Veröffentlichungstag der Anmeldung: 17.08.2005
(62) Teilanmeldung aus: 00962288.7
(73) Patentinhaber: LDR Medical SAS, 10430 Rosieres-Pres-Troyes (FR)
(72) Erfinder: Gau, Michel, 11590 Ouveillan (FR)
(74) Vertreter: Debay, Yves

(56) Entgegenhaltungen:
- EP-A- 0 577 179
- EP-A- 0 621 020
- DE-A1- 4 409 836
- DE-U1- 9 304 368

## Beschreibung

Die Erfindung betrifft eine Zwischenwirbel-Nucleus-Prothese, deren Aufgabe es ist, die Beweglichkeit im Bereich einer Bandscheibe wieder herzustellen. Diese Beweglichkeit wird physiologisch durch den Nucleus erzeugt, der als mechanisches Gelenkelement der Wirbelkörper angesehen werden kann.

Physiologisch wird der Nucleus von einem im wesentlichen sphärischen, nicht dehnbaren, jedoch verformbaren Körper gebildet, der wie eine Kugel zwischen zwei Wirbelendflachen eingesetzt ist, und zwar im wesentlichen in deren Zentrum, und der Bewegungen in Form der Neigung, der Rotation und der Verschiebung ermöglicht.

Die Wirbelendflachen werden ihrerseits von konzentrischen Faserlagen umgeben, die als Annulus bezeichnet werden und die Fasern enthalten, die von einer Schicht zur anderen sich schräg überkreuzen.

In dieser Anordnung bewegt sich der Nucleus, der eigentlich eine verformbare, jedoch nicht dehnbare Kapsel darstellt, die mit einer hydrophilen, geleeartigen Substanz gefüllt ist (Mucopolysaccharide) bei der Beugung der Wirbelsäule nach vorne oder nach hinten selbst nach hinten beziehungsweise nach vorn, wobei diese Bewegung des Nucleus durch die hinteren und die vorderen Fasern des Annulus und durch unterschiedliche Längsbänder begrenzt wird.

Bei einer seitlichen Neigung der Wirbelsäule dehnt die Verlagerung des Nucleus die Fasern auf der konvexen Seite. Die Verlagerung wird dabei durch die Zwischenwirbelbänder begrenzt.

Bei einer Drehung der Wirbelsäule wird die Bandscheibe mit Scherkräften beaufschlagt.

Der Nucleus, der einen hydrophilen Körper darstellt, hat im Ruhezustand einen bestimmten osmotischen Druck. Bei Belastung verliert der Nucleus Wasser. Die Dicke der Bandscheibe nimmt ab, sobald der Druck wieder reduziert wird, tritt Rehydrierung ein.

Die pathologischen Zustände, die die wechselseitigen Bewegungen der verschiedenen Wirbelkörper stören, sind dabei im wesentlichen Überlastung oder zu starke und zu häufige Belastungen oder natürliche Alterungsprozesse.

Bei einer zu starken, zu häufigen oder zu lang anhaltenden Belastung tritt die Rehydrierung nicht ein und damit kann der Druck, der vom Nucleus auf die die verschiedenen konzentrischen Lagen des Annulus bildenden Fasern ausübt, nicht mehr aufgebaut werden, damit spielt auch dieser Druck nicht mehr die Rolle der Rückstellung bei den Bewegungen. In diesem Falle tritt eine Instabilität auf und eine begleitende Arthrose.

Es ist Aufgabe der Erfindung, die Entwicklung dieser Instabilität dadurch zu verhindern, daß eine Nucleus-Prothese eingesetzt wird, deren Implantation in vivo erfolgen kann und mit weniger Traumatisierung als dies bei bekannten Prothesen dieser Art bisher möglich war.

Diese bekannten Prothesen, deren Prinzipien nachstehend untersucht werden, zeigen Nachteile, die man verstehen kann, wenn man die gegenseitigen Bewegungen und die Rolle des Nucleus bei diesen Bewegungen kennt.

Diese Nachteile treten auf sowohl bei der Benutzung als auch beim Einsetzen dieser bekannten Implantate.

Zunächst ist einfach vorgeschlagen worden, die Bandscheiben zu entfernen, die sich in dem traumatisierten Bereich befinden. Dann hat man einen knöchernen Pfropfen eingesetzt, teilweise mit und teilweise ohne Osteosynthese.

Die dadurch erzielte Blockierung von zwei benachbarten Wirbelkörpern hat jedoch nur die Belastungen auf die Bandscheiben der unmittelbar benachbarten Wirbelkörper übertragen, die dadurch übermäßig belastet und schnell geschädigt wurden, das Problem ist also nur verlagert und nicht gelöst worden.

Man hat weiterhin eine vollständige Bandscheibenprothese vorgeschlagen, die sowohl Annulus als auch Nucleus umfaßt.

Diese Praxis hat den Hauptnachteil, daß dazu eine vollständige Entfernung der geschädigten Bandscheibe notwendig ist und daß die Totalprothese dann zwischen zwei benachbarte Wirbelkörper eingeführt werden muß.

Für beide Phasen dieses Eingriffes muß man die gemeinsamen Wirbelkörperlängsbänder durchschneiden, nämlich das vordere Längsband (LVCA) und das hintere Längsband (LVCP), so daß man die natürlichen Bewegungen der Wirbelsäule nicht wieder herstellen kann, diese büßt somit an Zuverlässigkeit ein.

Um diese Nachteile zu vermeiden, wird im Rahmen der vorliegenden Erfindung davon ausgegangen, daß eine Nucleus-Prothese eingesetzt wird, ohne daß man die gesamte Bandscheibe reseziert. Auf diese Weise läßt sich durch das Ersetzen des Nucleus bereits die Entwicklung der Instabilität begrenzen, die ursächlich für die Verschlechterung ist.

Die Nucleus-Prothese erlaubt ohne Verletzung des Annulus oder der Längsbänder (LVCA und LVCP) durch die Verschiebung des ersetzten Nucleus auf den Annulus Druck auszuüben und ihn daher bei der Bewegung der Wirbelsäule so zu spannen, daß er die Gleichgewichtslage wiederherstellt.

Das Rotationszentrum, das auf diese Weise wiederhergestellt worden ist, bleibt tatsächlich beweglich und in der Lage, sich an die verschiedenen Beugebewegungen nach vorne und nach hinten, an Streckbewegungen und seitliche Neigungsbewegungen anzupassen.

Das Dokument EP 0 621 020 offenbart eine Zwischenwirbei-Nucleus-Prothese als Ersatz für einen Teil des kernbereichs einer Bandscheibe, geeignet zur Übertragung von Druckkräften, die aus mindestens drei beweglichen, insbesondere kugelförmigen, Körpern aus einem nicht oxidierenden Material besteht, deren Volumen an das des biologischen Nucleus angepaßt ist.

Die Nucleus-Prothese der vorliegenden Erfindung ist in Anspruch 1 definiert. Diese Prothese kann in dem Volumen untergebracht werden, welches nach der Entfernung des Nucleus zwischen zwei Wirbelendflächen frei wird.
Die Figur 1 zeigt eine Draufsicht auf einen Wirbelkörper mit den jeweiligen Positionen des Nucleus, des Annulus und der vorderen und hinteren Längsbänder.
Die Figur 2 ist eine perspektivische Ansicht eines Wirbelkörpers und zeigt den Annulus und den von ihm umgebenen Nucleus, der sich am Wirbelkörper abstützt.
Die Figur 3 ist eine perspektivische schematische Ansicht eines diametral geschnittenen Annulus und zeigt die konzentrischen, sich kreuzenden Faserlagen.

Die Figuren 4 und 5 sind schematische Seitenansichten von zwei benachbarten Wirbelkörpern bei einer Beugebewegung nach vorne beziehungsweise hinten.

Die Figur 6 ist eine schematische Vorderansicht von zwei benachbarten Wirbelkörpern während einer seitlichen Beugung der Wirbelsäule.

Die Figur 7 ist eine Draufsicht auf einen Wirbelkörper bei einer Rotationsbewegung.

Die Figuren 8, 9 und 10 sind schematische Ansichten von der Seite, von oben und von vorne einer Nucleus- Prothese mit nur einer Kugel, nicht gemäß der vorliegenden Erfindung.

Die Figur 11 ist eine schematische, beispielshafte Längsschnittansicht längs Linie A-A in Figur 8.

Die Figuren 12, 13 und 14 sind schematische Ansichten von oben, von vorne und von der Seite einer Nucleus-Prothese mit mehreren Kugeln.

Die Figuren 15, 16 und 17 sind schematische Ansichten von oben, von vorne und von der Seite einer Nucleus-Prothese mit einer Dampfungseinrichtung.

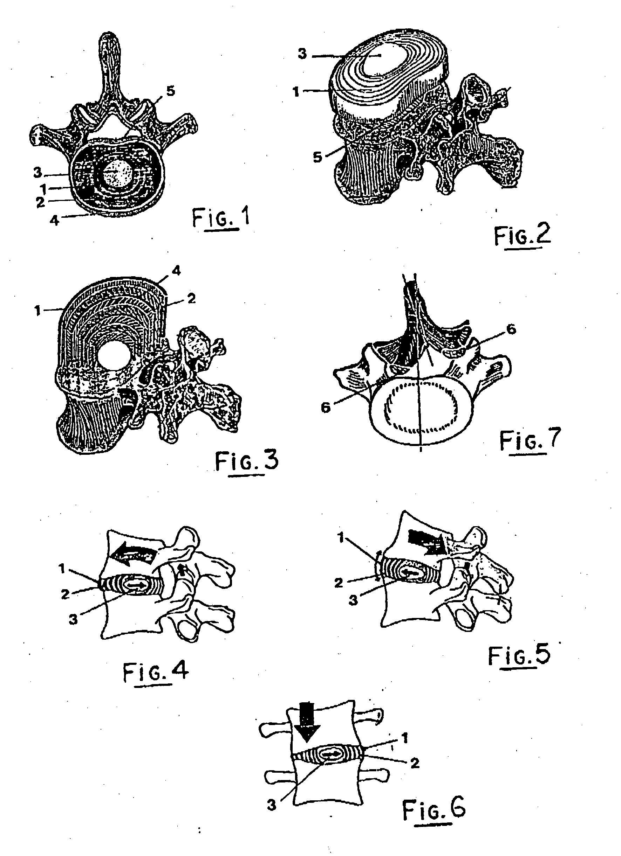

Die Figuren 1 und 2 zeigen gut eine Zwischenwirbelebene, die gebildet wird durch eine Vielzahl von konzentrischen Lagen von sich kreuzenden Fasern 1 und 2, deutlich sichtbar in Figur 3, und in deren Zentrum jeweils der Nucleus 3 angeordnet ist, während an der Außenseite das vordere Längsband (LVCA) 4 und das hintere Längsband (LVCP) 5 angeordnet sind.

Die Figuren 4 und 5 zeigen die Kompressionskräfte, die durch den Nucleus 3 auf die konzentrischen Annuluslagen ausgeübt werden, und zwar jeweils an der Außenseite des Biegewinkels bei der Biegung nach vorne oder nach hinten.

Der Nucleus 3 übt in gleicher Weise einen Druck auf die Ringe des Annulus aus, und zwar auf der Außenseite des seit1ichen Neigungswinkels (Figur 6).

Jede dieser Verschiebungen des Nucleus 3 stellt eine Rückkehr in die Gleichgewichtslage sicher.

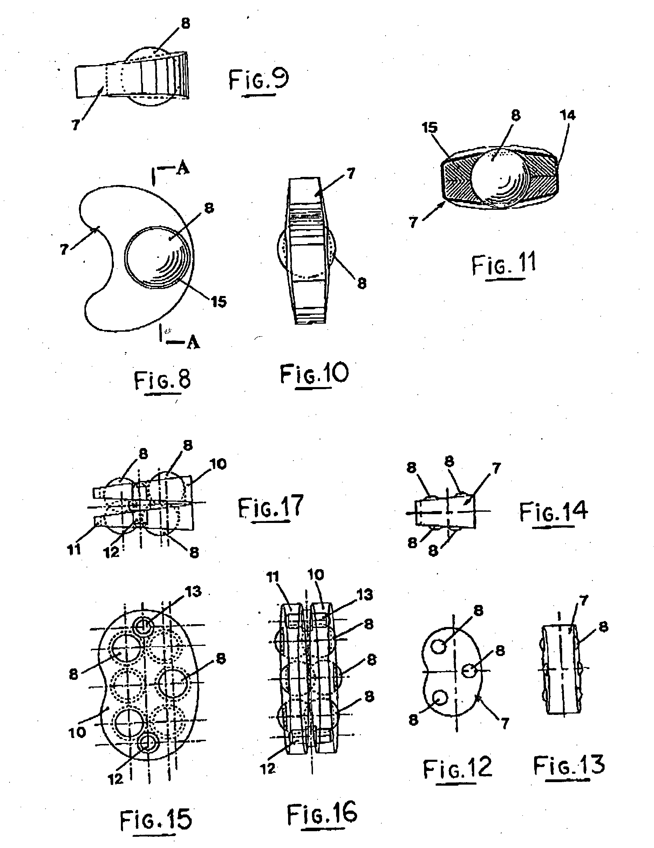

Bei einer Rotation in einer horizontalen Ebene sind es die Gelenkkapseln 6, die längs eines fiktiven Kreises an der Außenseite liegen, die die kreisförmige Verlagerung ermöglichen und die die Rotationsbewegung begrenzen (Figur 7), der Nucleus 3 wird dabei mit Scherkräften beaufschlagt.

Der Nucleus spielt also in all diesen Fällen die Rolle eines Bewegungsdämpfers und stellt bei dieser Bewegung die Rückkehr in die Gleichgewichtslage sicher. Man erkennt daraus die Folgen, die sich einstellen, wenn der Nucleus verändert wird.

Man erkennt daraus auch, daß eine Wiederherstellung der gegenseitigen Bewegungen benachbarter Wirbelkörper bereits dadurch erreicht werden kann, daß man eine Nucleus-Prothese einsetzt, deren Implantation einen sehr großen chirurgischen Vorteil bedeutet.

Bei den bestehenden operativen Techniken führt die chirurgische Wirbelkörperverschmelzung zu einer dauerhaften Fusion von benachbarten Wirbeln, die zu beiden Seiten der geschädigten Bandscheibe angeordnet sind. Zu diesem Zweck wird die Bandscheibe vollständig entfernt.

Wenn die Fusion von zwei Wirbelkörpern durch Arthrodese gelingt, dann werden die Probleme der Degenerierung auf die benachbarten Bandscheiben (oben oder unten) über- tragen, wobei diese Probleme noch durch die Festigkeit des auf diese Weise erzeugten Wirbelblocks erhöht werden.

Wenn jedoch die Fusion nicht gelingt, was der Grund für häufige Fehlschläge ist und was zu einer Fortdauer der Beschwerden führt, dann kann man häufig nur sehr schwierig nachoperieren.

Im Fall der Implantation einer Totalprothese der Bandscheibe hat man festgestellt, daß diese ein kompliziertes und schwieriges chirurgisches Verfahren notwendig macht, wobei man eine große Öffnung benötigt, um die natürliche Bandscheibe entfernen und die Totalprothese einsetzen zu können.

Dazu gehört die Notwendigkeit der Durchtrennung und der Verlagerung großer Bauchgefäße (Aorta und Vene) und des Nervenbündels der Sexualorgane.

Dabei ergibt sich das Risiko großer Gefäßblutungen, die nur schwer zu kontrollieren sind.

Dies kann auch zu schwerwiegenden Sexualproblemen führen: Impotenz und retrograde Ejakulation.

Darüber hinaus führt die Totalresektion der Bandscheibe zu einer Zerstörung des gesamten Annulus und der vorderen und hinteren Zwischenwirbellängsbänder 4 und 5 und erzeugt dadurch schwerwiegende Instabilitätsrisiken.

Außerdem gibt es in diesem Falle keinerlei Rückkehr, es bleibt nur der Weg einer Wirbelfusion mit den oben beschriebenen Nachteilen.

Die erfindungsgemäße Nucleus-Prothese weist keinen dieser Nachteile auf. Sie ermöglicht es, den geschädigten Nucleus sowohl der Form als auch der Funktion nach zu ersetzen. Sie ist geeignet, die Degeneration der Bandscheibe dadurch zu beenden, daß sie diese wieder stabilisiert.

Das chirurgische Implantationsverfahren ist außerordentlich einfach.

Zunächst kann die Implantation endoskopisch durchgeführt werden. Sie ist schnell und schonend.

Im Falle eines Fehlschlages kann man durch Entfernen der Nucleus-Prothese den ursprünglichen Zustand wieder herstellen.

Sie ist nicht mit Gefäßrisiken verbunden und auch nicht mit Risiken für die Sexualorgane.

Es genügt tatsächlich, endoskopisch eine Öffnung zwischen zwei Wirbelkörper durch den Annulus hindurch einzubringen, die gerade groß genug ist, um den geschädigten Nucleus zu erreichen und zu entfernen und um anschließend auf demselben Wege den künstlichen Nucleus einzuführen, der sich automatisch in der ursprünglichen Kammer zentriert, anschließend wird die erzeugte Öffnung durch eine Naht verschlossen.

Unabhängig davon, daß dieser Eingriff schnell erfolgt, kann der Faserk6rper, der nur geringfügig durch den Einschnitt geschädigt ist, sich spontan und schnell und praktisch schmerzfrei erholen. Die vorderen und hinteren Längsbänder werden in keiner Weise traumatisiert, so daß man die ursprüngliche Beweglichkeit der Wirbelsäule wiederherstellen kann.

Die einfache Implantation dieser Nucleus-Prothese und die Tatsache, daß bei diesem Eingriff keinerlei Risiken auftreten, führt dazu, daß ihre Anwendung immer dann angezeigt ist, wenn primäre oder sekundäre Bandscheibenschädigungen eingetreten sind, und zwar bereits beim ersten Auftreten der Krankheit, bevor definitive Schädigungen des Knochens und der Gelenke auftreten.

Die Nucleus-Prothese besteht im wesentlichen aus mindestens einem beweglichen Körper 8, aus einem festen, nicht oxidierenden, biokompatiblen Material, welcher sich in zwei Achsen einer Ebene bewegen kann (beispielsweise eine nichtrostende Stahlkugel oder eine Kugel aus Titan), deren Volumen angepaßt ist an das des biologischen Nucleus. Dieser bewegliche Körper oder diese Kugel B ist in einem im folgenden als Käfig be- zeichneten Körper 7 gelagert, der ein Gehäuse 14 umfaßt (beispielshaft in Figur 11 dargestellt), welches aus einem leichten, festen, nicht oxidierenden und biokompatiblen Material besteht (beispielsweise Titan), wobei dieses Gehäuse 14 eine Masse 15 enthält aus einem Material mit einem minimalen Reibungskoeffizienten (beispielsweise Polyethylen). Im inneren befindet sich ein Aufnahmeraum für den beweglichen Körper oder die Kugel 8, diese ist in diesem Aufnahmeraum gefangen, je- doch frei um ihren Mittelpunkt verdrehbar und derart gelagert, daß sie auf beiden gegenüberliegenden Seiten (Oberseite und Unterseite) des Käfigs 7 in Form einer Kugelkalotte austritt, deren Höhe etwa 1/10 des Durchmessers des beweglichen Körpers oder der Kugel 8 entspricht, wobei dieser Wert nicht streng eingehalten werden muß.

Das Volumen des Käfigs 7, der den mobilen Körper 8 aufnimmt, ist seinerseits möglichst eng an das Volumen des biologischen Nucleus angepaßt, wobei natürlich berücksichtigt ist, daß der Käfig 7 als Lagerung für die Kugel 8 dienen muß. Dadurch kann sich die Prothese selbst positionieren, es ist ihr also möglich, sich immer in der anatomischen Position zu befinden und dadurch die natürliche Bewegung zwischen zwei Wirbelkörpern wiederherzustellen.

Der die Kugel 8 lagernde Käfig 7 kann in der Draufsicht (Figur 8) eine mehr oder weniger gebogene Form aufweisen, die symmetrisch im Bezug auf eine transversale Mittelebene ist. Im Querschnitt (Figur 10) weist der Käfig vorzugsweise die Form eines Trapezes auf, dessen kleine Stirnfläche die Enden der mehr oder weniger stark gebogenen Form aufnimmt.

Diese trapezförmige, von vorne nach hinten orientierte Asymmetrie des Käfigs 7 führt dazu, daß eine Drehung desselben zwischen den zwei Wirbelkörpern vermieden wird, wenn sich die in dieser Weise gebildete, den künstlichen Nucleus (Kugel 8) aufnehmende Prothese verschiebt.

Bei einer in den Figuren 12, 13 und 14 dargestellten abgewandelten Ausführungsform kann der Käfig 7, der im Querschnitt im wesentlichen die Form eines gleichschenkligen Trapezes aufweist (Figur 13), mehrere identische Kugeln 8 (Figur 14) aufnehmen, die an der Außenseite eine gemeinsame fiktive Ebene berühren, die auf beiden Seiten der horizontalen Mittelebene des Käfigs angeordnet sind. Die Kugeln befinden sich auf beiden Seiten des Käfigs 7 an den drei Eckpunkten eines gleichschenkligen Dreiecks.

Um den Komfort zu erhöhen, kann der Käfig 7 entsprechend den Ausführungsformen der Figuren 15, 16 und 17 aus zwei identischen Einzelkörpern 10, 11 bestehen, die test, nicht oxidierbar und biokompatibel ausgebildet sind und die Form eines gleichschenkligen Trapezes aufweisen. Sie sind so angeordnet, daß ihre senkrecht auf den parallelen Basisflächen stehenden, längs deren Mittellinien verlaufenden Mittelebenen im wesentlichen parallel zueinander verlaufen und daß die großen Basisflächen der trapezförmigen Einzelkörper 10 und 11 im wesentlichen in derselben senkrechten Ebene liegen.

Die zwei auf diese Weise ausgebildeten Einzelkörper sind untereinander durch zwei elastische Lagerelemente 12 und 13 verbunden, die senkrecht zu den Mittelebenen der Einzelkörper 10 und 11 verlaufen und nahe dem Ende einer großen Achse angeordnet sind, die ihrerseits eine gemeinsame Tangente an die Sätze der Kugeln 8 ausbildet, die in jeder Außenseite der Gesamtanordnung so angeordnet sind, daß die Kugeln an den drei Ecken eines gleichschenkligen Dreiecks angeordnet sind, die ihrerseits auf gegenüberliegenden Seiten der Anordnung in entgegengesetzte Richtung weisen.

Wenn eine Prothese entsprechend den Figuren 8 bis 17 eingesetzt ist, dann ist verständlich, daß bereits unmittelbar nach dem Einführen in den Hohlraum des Nucleus die Prothese die Rolle des biologischen Nucleus übernimmt und die sich kreuzenden Fasern des Annulus bei einer seitlichen oder bei einer Bewegung nach vorne oder nach hinten unter Spannung setzt und dadurch eine Rückkehr in die Gleichgewichtslage erzeugt. Der schnelle Eingriff, der zum Einsetzen notwendig ist, vermeidet jegliche Traumatisierungsrisiken und führt zu einer schnellen Heilung der einzig notwendigen Öffnung.

Die endgültige Auswahl der hier vorgeschlagenen Modelle, die alle dieselben Vorteile hinsichtlich des Einsetzens und hinsichtlich der physiologischen Resultate aufweisen, wird sich aufgrund der klinischen Resultate im Laufe der Zeit ergeben.

Selbstverständlich kann der Käfig 7, der eine Kugel 8 oder mehrere Kugeln 8 enthält und für den vorstehend mehrere Ausführungsformen vorgeschlagen worden sind, ohne Abkehr von den erfindungsgemäßen Gedanken auch andere äquivalente Formen aufweisen, es ist lediglich wichtig, daß er die Kugel oder die Kugeln 8 lagert, daß diese in ihm frei drehbar sind und daß sein Volumen an das Volumen angepaßt ist, welches nach Entfernung des biologischen Nucleus zur Verfügung steht.

Die beschriebene Nucleus-Prothese kann bei allen vorstehend beschriebenen Ausführungsformen spontan in der anatomischen Umgebung die eigene Position einnehmen, so daß alle natürlichen Bewegungen zwischen zwei Wirbelkörpern wiederhergestellt werden können (seitliche Neigung, Neigung nach vorne und nach hinten, Rotation). Die konische Form (im Querschnitt trapezförmig) des Käfigs 7 erleichtert die Verschiebung in der Ebene der Wirbelendflächen und verhindert gleichzeitig die Rotation des Käfigs um seine zentrale Achse, außerdem das Einsinken des Implantates in die Wirbelendflächen.

## Patentansprüche

1. Zwischenwirbel-Nucleus-Prothese, deren Volumen an das des biologischen Nucleus angepaßt ist, **dadurch gekennzeichnet, dass** sie einen Käfig (7) besitzt, der folgende Elemente aufweist:
- mehrere identische Kugeln (8) aus einem festen, nicht oxidierenden, biokompatiblen Material, die in zwei Achsen einer Ebene auf beiden Seiten einer horizontalen Mittelebene des Käfigs beweglich sind, um eine gleiche, fiktive, außerhalb des Käfigs liegenden Ebene zu berühren und die auf einer der Seiten des Käfigs (7) in der Form einer Kugelkalotte hervorstehen;
- eine Vielzahl von Hohlräumen, die das Einschließen jeweils einer Kugel (8) ermöglicht die frei drehbar um ihren Mittelpunkt gelagert ist.

2. Prothese nach Anspruch 1, bei welcher die Kugeln (8) unverschiebbar in dem jeweiligen Hohlraum gelagert sind.

3. Prothese nach Anspruch 1 oder 2, bei welcher der Käfig (7) aus zwei identischen, gleichschenkligen trapezförmigen Einzelkörpecn (10, 11) gebildet ist.

4. Prothese nach einem der Ansprüche 1 bis 3, bei welcher der mit Kugeln (8) versehene Käfig ein Volumen aufweist, der unter Berücksichtigung seiner Aufgabe als Lagerung der Kugeln so gut wie möglich dem Volumen, des biologischen Nucleus angepaßt ist, so dass damit eine Selbstpositionierung der Prothese gesichert ist, durch die sich diese immer in der anatomischen Position befindet, um die natürlichen Bewegungen zwischen zwei Wirbelkörpern wiederherzustellen.

## Claims

1. Intervertebral nucleus prosthesis, the volume of which is matched to that of the biological nucleus, **characterised in that** it has a cage (7) which exhibits the following elements:
- a plurality of identical balls (8) which are made of a solid, non-oxidising, biocompatible material and are moveable in two axes of a plane on both sides of a horizontal median plane of the cage in order to contact a same, imaginary plane lying outside the cage and which protrude on one of the sides of the cage (7) in the form of a spherical calotte;
- a plurality of cavities which each allow the inclusion of a ball (8) which is mounted so as to be able to turn freely about its centre point.

2. Prosthesis according to claim 1, in which the balls (8) are mounted so that they cannot be displaced in the respective cavity.

3. Prosthesis according to claim 1 or 2, in which the cage (7) is formed of two identical, isoceles, trapezoidal, individual bodies (10, 11).

4. Prosthesis according to one of claims 1 to 3, in which the cage provided with balls (8) exhibits a volume which allowing for its function as mounting for the balls is matched as well as possible to the volume of the biological nucleus so that as a result self-positioning of the prosthesis is assured so that this is always in the anatomical position in order to restore the natural movements between two vertebral bodies.

## Revendications

1. Prothèse de nucléus de disque intervertébral, dont le volume est ajusté à celui du nucléus biologique, **caractérisée en ce qu'**elle est pourvue d'une cage (7) qui comporte les éléments suivants :
- plusieurs billes (8) identiques en un matériau résistant, inoxydable, biocompatible, mobiles sur deux axes d'un plan de part et d'autre d'un plan médian horizontal de la cage pour contacter un plan fiictif équivalent situé à l'extérieur de la cage, et qui font saillie sous la forme d'une calotte sphérique sur un des côtés de la cage (7) ;
- une pluralité de cavités permettant chacune l'inclusion d'une bille (8) respective, logée de manière à être librement rotative autour de son centre.

2. Prothèse selon la revendication 1, où les billes (8) sont logées de manière à être fixes dans leur cavité respective.

3. Prothèse selon la revendication 1 ou 2, où la cage (7) est constituée de deux corps individuels (10, 11) identiques de forme trapézoïdale isocèle.

4. Prothèse selon l'une des revendications 1 à 3, où, en tenant compte de sa fonction de logement des billes, la cage pourvue de billes (8) présente un volume ajusté de manière aussi satisfaisante que possible au volume du nucléus biologique, de manière à assurer ainsi une mise en place spontanée de la prothèse par laquelle celle-ci se trouve toujours en position anatomique pour rétablir les mouvements naturels entre deux corps de vertèbres.
